# EUROPÄISCHE PATENTANMELDUNG

(11) **EP 2 093 219 A1**
(43) Veröffentlichungstag der Anmeldung: **26.08.2009**
(21) Anmeldenummer: 08151842.5
(22) Anmeldetag: 22.02.2008
(51) Int. Cl.: C07D 265/18, A61P 11/00

(54) **Kristalline, enantiomerenreine Salzform eines Betamimetikums und dessen Verwendung als Arzneimittel**

(71) Anmelder: BOEHRINGER INGELHEIM INTERNATIONAL GMBH, 55216 Ingelheim (DE)
(72) Erfinder: Die Erfindernennung liegt noch nicht vor
(74) Vertreter: Hammann, Heinz

(57) **Zusammenfassung**

Die vorliegende Erfindung betrifft ein kristallines, enantiomerenreines Hydrochlorid-Salz von N-(5-{2-[3-(4,4-diethyl-2-oxo-4H-benzo[d][1,3]oxazin-1-yl)-1,1-dimethyl-propylamino]-1-hydroxy-ethyl}-2-hydroxy-phenyl)-methansulfonamide, bevorzugt von N-(5-{(R)-2-[3-(4,4-diethyl-2-oxo-4H-benzo[d][1,3]oxazin-1-yl)-1,1-dimethyl-propylamino]-1-hydroxyethyl}-2-hydroxy-phenyl)-methansulfonamide und dessen Wirkung als langwirksames Betamimetikum alleine oder in Kombination mit einem oder mehreren, weiteren Wirkstoffen zur Behandlung von Atemwegserkrankungen.

## Beschreibung

Die vorliegende Erfindung betrifft ein kristallines, enantiomerenreines Hydrochlorid-Salz von N-(5-{(R)-2-[3-(4,4-Diethyl-2-oxo-4H-benzo[d][1,3]oxazin-1-yl)-1,1-dimethylpropylamino]-1-hydroxy-ethyl}-2-hydroxy-phenyl)-methansulfonamid mit einem Schmelzpunkt von 215°C und der Struktur 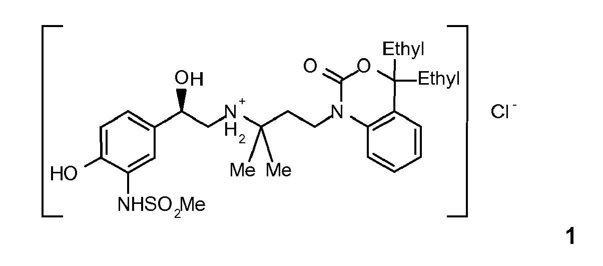 und dessen Wirkung als langwirksames Betamimetikum alleine oder in Kombination mit einem oder mehreren, weiteren Wirkstoffen zur Behandlung von Atemwegserkrankungen.

### HINTERGRUND DER ERFINDUNG

Betamimetika (ß-adrenerge Substanzen) sind aus dem Stand der Technik bekannt. Beispielsweise sei diesbezüglich auf die Offenbarung der US 4,460,581 verwiesen, die Betamimetika zur Therapie unterschiedlichster Erkrankungen vorschlägt.

Zur medikamentösen Therapie von Erkrankungen ist es häufig wünschenswert, Arzneimittel mit einer längeren Wirkungsdauer bereitzustellen. Hierdurch kann in der Regel gewährleistet werden, dass die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei.

Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, dass der Patient sich relativ schnell an die regelmäßige Einnahme des Medikaments zu bestimmten Tageszeiten gewöhnen kann.

Die enantiomerenreine Verbindung gemäß Formel 1 stellt ein langwirksames Betamimetikum dar.

Die Applikation von 1 bei der Verwendung als Medikament zur Behandlung von Atemwegserkrankungen erfolgt vorzugsweise auf inhalativem Wege. Hierbei können geeignete Inhalationspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten.

Die richtige Herstellung der vorgenannten, zur inhalativen Verabreichung eines Arzneiwirkstoffes verwendbaren Zusammensetzungen stützt sich auf verschiedene Parameter, die mit der Beschaffenheit des Arzneiwirkstoffes selbst verbunden sind. Ohne Einschränkung darauf sind Beispiele dieser Parameter die Wirkstabilität des Ausgangsstoffes unter verschiedenen Umgebungsbedingungen, die Stabilität im Verlauf der Herstellung der pharmazeutischen Formulierung sowie die Stabilität in den Endzusammensetzungen des Arzneimittels. Der zur Herstellung der vorgenannten Arzneimittelzusammensetzungen verwendete Arzneiwirkstoff sollte so rein wie möglich sein und seine Stabilität bei Langzeitlagerung muss unter verschiedenen Umgebungsbedingungen gewährleistet sein. Dies ist zwingend erforderlich, um zu verhindern, dass Arzneimittelzusammensetzungen Verwendung finden, in denen neben tatsächlichem Wirkstoff beispielsweise Abbauprodukte desselben enthalten sind. In einem solchen Fall könnte ein in Kapseln vorgefundener Gehalt an Wirkstoff niedriger sein als spezifiziert.

Die Absorption von Feuchtigkeit vermindert den Gehalt an Arzneiwirkstoff wegen der durch die Wasseraufnahme verursachten Gewichtszunahme. Zur Aufnahme von Feuchtigkeit neigende Arzneimittel müssen während der Lagerung vor Feuchtigkeit geschützt werden, beispielsweise durch Zusatz von geeigneten Trockenmitteln oder durch Lagerung des Arzneimittels in einer vor Feuchtigkeit geschützten Umgebung. Zudem kann die Aufnahme von Feuchtigkeit den Gehalt an Arzneiwirkstoff während der Herstellung vermindern, wenn das Arzneimittel der Umgebung ohne jeglichen Schutz vor Feuchtigkeit ausgesetzt wird.

Eine gleichmäßige Verteilung des Arzneimittels in der Formulierung ist ebenfalls ein kritischer Faktor, insbesondere wenn eine niedrige Dosierung des Arzneimittels erforderlich ist. Um die gleichmäßige Verteilung sicherzustellen, kann man die Teilchengröße des Wirkstoffes auf einen geeigneten Wert vermindern, beispielsweise durch Mahlen. Ein weiterer Aspekt, der bei inhalativ, mittels eines Pulvers zu applizierenden Wirkstoffen bedeutsam ist, ist durch den Umstand bedingt, dass nur Teilchen einer bestimmten Teilchengröße bei der Inhalation in die Lunge gelangen. Die Teilchengröße dieser lungengängigen Partikel (inhalierbarer Anteil) liegt im Bereich zwischen 2 und 5 µm. Um Wirkstoffe mit entsprechender Teilchengröße zu erhalten, ist ebenfalls ein Mahlvorgang (so genanntes Mikronisieren) erforderlich.

Da als Begleiterscheinung des Mahlens (bzw. Mikronisieren) trotz der beim Verfahrensablaufs erforderlichen harten Bedingungen ein Abbau des Arzneimittelwirkstoffs weitestgehend vermieden werden muss, stellt eine hohe Stabilität des Wirkstoffs gegenüber dem Mahlvorgang eine unabdingbare Notwendigkeit dar. Nur eine ausreichend große Stabilität des Wirkstoffs beim Mahlvorgang erlaubt die Herstellung einer homogenen Arzneimittelformulierung, in der stets in reproduzierbarer Art und Weise die festgelegte Menge an Wirkstoff enthalten ist.

Eine spezifische Problematik, die beim Mahlvorgang zur Herstellung der gewünschten Arzneimittelformulierung auftreten kann, ist die durch diesen Prozess erfolgende Energiezufuhr und die Belastung der Oberfläche der Kristalle. Dies kann unter Umständen zu polymorphen Veränderungen, zu einer Umwandlung zur amorphen Gestalt hin oder zu einer Änderung des Kristallgitters führen. Da für die pharmazeutische Qualität einer Arzneimittelformulierung stets dieselbe kristalline Morphologie des Wirkstoffs gewährleistet sein muss, sind auch vor diesem Hintergrund an die Stabilität und Eigenschaften des kristallinen Wirkstoffs erhöhte Anforderungen zu stellen.

Als erfindungsgemäß besonders bedeutsamer Aspekt ist zu berücksichtigen, dass mit Zunahme der spezifischen Oberfläche, wie dies zwangsläufig im Rahmen der Mikronisierung auftritt, entsprechend auch die Oberflächenenergie eines partikulären Systems zunimmt. Da jedes System aus thermodynamischer Sicht zu einem Energieminimum strebt, ergibt sich für partikuläre Systeme, die eine inhalierbare Teilchengröße und damit eine spezifische Oberfläche von typischerweise zwischen 1m²/g bis 10m²/g aufweisen, dass hohe Anforderungen an die Kristallinität des mikronisierten Arzneimittelwirkstoffes gestellt werden. Es ist hierbei zu beachten, dass die kristalline Ordnung thermodynamisch bevorzugt gegenüber ungeordneten Systemen ist und somit kristalline partikuläre Systeme aus thermodynamischer Sicht physikalisch stabiler sind, im besonderen Maße, wenn die spezifische Kristallstruktur im Vergleich zu anderen Kristallstrukturen aus thermodynamischer Sicht besonders stabil ist.

Eine erfindungsgemäß zu lösende Aufgabe ist daher insbesondere die Bereitstellung einer thermodynamisch besonders stabilen Form der Verbindung der Formel 1.

### DETAILLIERTE BESCHREIBUNG DER ERFINDUNG

Es wurde nun gefunden, dass die vorstehend genannten Aufgaben durch eine kristalline, enantiomerenreine Verbindungen der Formel 1 gelöst werden. Gegenstand der vorliegenden Erfindung ist demnach die kristalline, enantiomerenreine Verbindung N-(5-{(R)-2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid mit der Struktur:

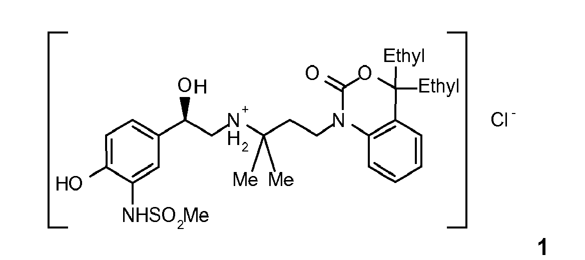

Die Bezeichnung enantiomerenrein beschreibt im Rahmen der vorliegenden Erfindung Verbindungen der Formel **1**, die in einer Enantiomerenreinheit von wenigstens 85%ee, bevorzugt von wenigstens 90%ee, besonders bevorzugt von ≥ 95%ee vorliegen. Die Bezeichnung ee (enantiomeric excess) ist im Stand der Technik bekannt und beschreibt den optischen Reinheitsgrad chiraler Verbindungen.

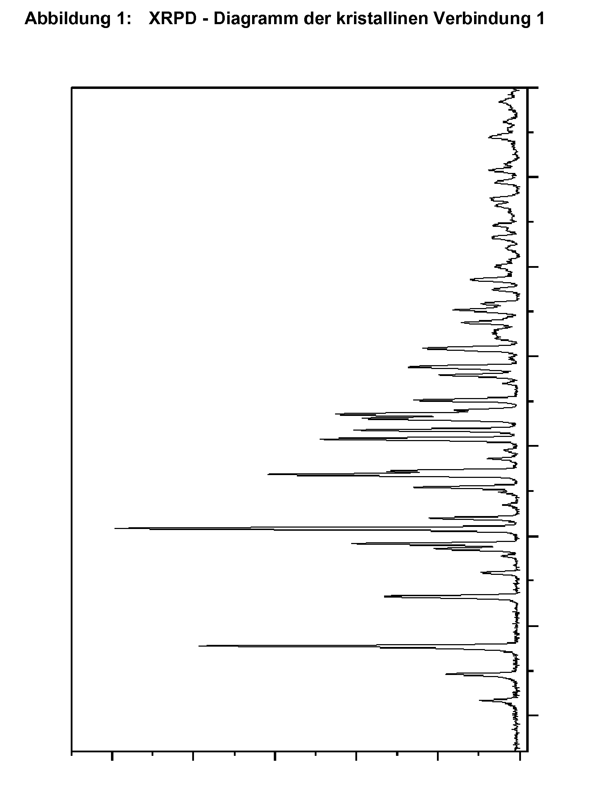

Die kristalline, enantiomerenreine Verbindung 1 kann dabei durch einen Schmelzpunkt von 215 ± 3°C charakterisiert werden. Bevorzugt erfolgt diese Charakterisierung mittels Thermoanalyse (DSC/TG). Weiterhin charakterisiert ist diese neue Form durch ein Röntgenpulver-diagramm (siehe Abbildung 1) mit charakteristischen Röntgenreflexen gemäß Tabelle 1.

**Table 1: Charakteristische X-ray Diffraction Peaks (bis 30 ° 2 Θ) basierend auf normalisierten Intensitätsangaben**

| 2 Θ | dₕₖₗ | I/Iₒ | | Indexing | | 2 Θ_{obs} - 2 Θ_{calc} |
|---|---|---|---|---|---|---|
| [°] | [Å] | | h | k | l | [°] |
| 5,77 | 15,30 | 9 | 2 | 0 | 0 | -0,005 |
| 7,25 | 12,19 | 18 | 1 | 1 | 0 | -0,002 |
| 8,81 | 10,03 | 81 | 2 | 1 | 0 | 0,002 |
| 11,57 | 7,64 | 33 | 4 | 0 | 0 | 0,003 |
| 12,90 | 6,86 | 9 | 1 | 0 | 1 | 0,003 |
| 13,85 | 6,39 | 4 | 2 | 0 | 1 | 0,010 |
| 14,22 | 6,22 | 21 | 0 | 1 | 1 | -0,003 |
| 14,52 | 6,10 | 42 | 1 | 1 | 1 | -0,003 |
| 15,37 | 5,76 | 100 | 2 | 1 | 1 | 0,001 |
| 15,94 | 5,56 | 22 | 3 | 2 | 0 | 0,030 |
| 16,69 | 5,31 | 4 | 3 | 1 | 1 | 0,003 |
| 17,39 | 5,09 | 4 | 6 | 0 | 0 | 0,008 |
| 17,67 | 5,02 | 26 | 4 | 2 | 0 | 0,000 |
| 18,38 | 4,82 | 63 | 0 | 2 | 1 | 0,036 |
| 18,56 | 4,78 | 32 | 1 | 2 | 1 | -0,010 |
| 19,25 | 4,61 | 7 | 2 | 2 | 1 | 0,003 |
| 19,70 | 4,50 | 3 | 5 | 2 | 0 | -0,009 |
| 20,35 | 4,36 | 50 | 3 | 2 | 1 | 0,027 |
| 20,86 | 4,26 | 41 | 2 | 3 | 0 | 0,000 |
| 21,50 | 4,13 | 39 | 6 | 0 | 1 | -0,008 |
| 21,74 | 4,09 | 45 | 4 | 2 | 1 | -0,001 |
| 21,95 | 4,05 | 15 | 6 | 2 | 0 | -0,008 |
| 22,53 | 3,94 | 26 | 6 | 1 | 1 | -0,006 |
| 23,44 | 3,79 | 3 | 5 | 2 | 1 | -0,004 |
| 23,89 | 3,72 | 19 | 1 | 3 | 1 | -0,001 |
| 24,36 | 3,65 | 27 | 7 | 2 | 0 | -0,001 |
| 24,88 | 3,58 | 2 | 7 | 1 | 1 | -0,009 |
| 25,39 | 3,51 | 24 | 6 | 2 | 1 | 0,013 |
| 25,95 | 3,43 | 6 | 2 | 0 | 2 | -0,003 |
| 26,15 | 3,40 | 5 | 0 | 1 | 2 | -0,016 |
| 26,82 | 3,32 | 14 | 0 | 4 | 0 | 0,009 |
| 27,53 | 3,24 | 16 | 7 | 2 | 1 | 0,030 |
| 27,89 | 3,20 | 9 | 4 | 0 | 2 | 0,003 |
| 28,68 | 3,11 | 6 | 7 | 3 | 0 | 0,005 |
| 29,22 | 3,05 | 12 | 5 | 0 | 2 | -0,030 |
| 29,97 | 2,98 | 6 | 9 | 1 | 1 | 0,028 |

Die kristalline, enantiomerenreine Verbindung **1** kann dabei durch ein Röntgenpulverdiagramm mit den charakteristischen Röntgenreflexen bei d = 10,03 Å; 5,76 Å; 4,82 Å; 4,36 Å; inter alia, charakterisiert werden. Besonders bevorzugt ist eine kristalline, enantiomerenreine Verbindung **1** mit den charakteristischen Röntgenreflexen bei d = 10,03 Å; 6,10 Å; 5,76 Å; 4,82 Å; 4,36 Å; 4,26 Å; 4,13 Å; 4,09 Å; inter alia.

Das Röntgenpulverdiagramm der kristallinen, enantiomerenreinen Verbindung **1** lässt sich mit folgender orthorhombischen Zelle (Raumgruppe P2₁2₁2₁) mit folgenden Zellkonstanten: a = 30.583(16) Å, b = 13.291(8) Å, c= 7.040(3) Å, α = β = γ = 90 °, V = 2861.6(37) Å³ (Indexwert 107,3) indizieren.

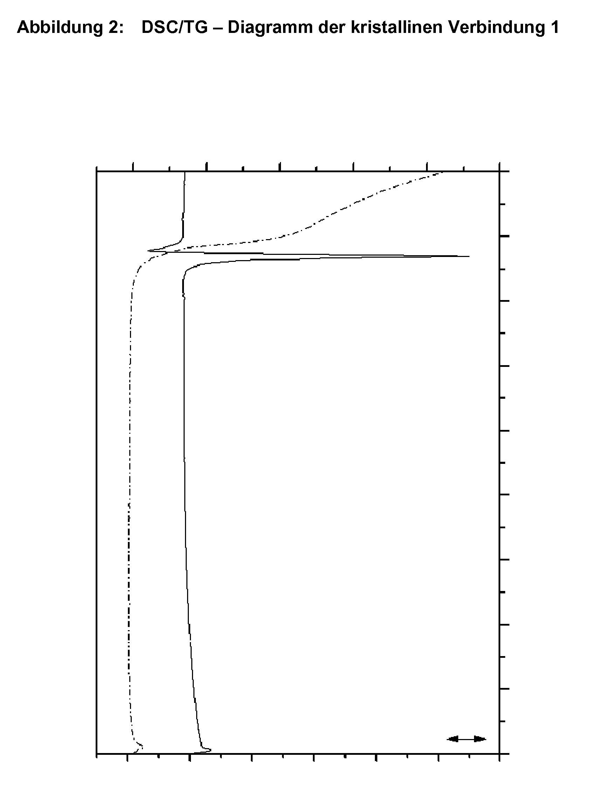

Bevorzugt ist die kristalline, enantiomerenreine Verbindung **1** dadurch gekennzeichnet, dass sie bei thermischer Belastung bis 210°C thermisch stabil ist (siehe Abbildung 2).

Stärkster Gewichtsverlust im TG-Experiment wird beim Aufschmelzen der Substanz beobachtet (Schmelzen unter Zersetzung). Der Trocknungsverlust bis 210°C umfasst ausschließlich an der Oberfläche frei verfügbares / oberflächlich adsorbiertes Wasser. Ausgehend von diesem thermischen Verhalten im TG-Experiment kann auf die Stöchiometrie der kristallinen Verbindung geschlossen werden, die folglich nicht als Solvat bzw. Hydrat vorliegt.

Die kristalline, enantiomerenreine Verbindung **1** zeichnet sich darüberhinaus noch durch folgendes Wassersorptionsverhalten aus: Im Luftfeuchtebereich zwischen 10 - 90 % n.F. nimmt **1** weniger als 0,5 Gew.% Wasser auf.

Weiterer Gegenstand der vorliegenden Erfindung sind pharmazeutische Zusammensetzungen, dadurch gekennzeichnet dass sie die erfindungsgemäße kristalline, enantiomerenreine Verbindung **1** enthalten. Bevorzugt werden diese Zusammensetzungen zur Behandlung von Atemwegserkrankungen eingesetzt. Die vorliegende Erfindung betrifft ferner die Verwendung der kristallinen, enantiomerenreinen Verbindung **1** zur Herstellung eines Arzneimittels zur Behandlung von Atemwegserkrankungen.

Die vorliegende Erfindung betrifft bevorzugt die Verwendung der vorstehend genannten kristallinen und enantiomerenreinen Verbindungen der Formel **1** zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

### INDIKATIONEN

Bevorzugt ist die Verwendung der kristallinen und enantiomerenreinen Verbindung der Formel **1** zur Herstellung eines Arzneimittels zur Behandlung von Obstruktive Lungenerkrankungen, die ausgewählt sind aus der Gruppe bestehend aus COPD (chronisch obstruktive pulmonale Erkrankung), Asthma Bronchiale, pädiatrisches Asthma, schweres Asthma, akuter Asthma-Anfall und chronische Bronchitis, wobei die Verwendung zur Herstellung eines Arzneimittels zur Behandlung von Asthma Bronchiale erfindungsgemäß besonders bevorzugt ist.

Bevorzugt ist ferner die Verwendung der kristallinen und enantiomerenreinen Verbindung der Formel **1** zur Herstellung eines Arzneimittels zur Behandlung von Lungenemphysemen, die ihren Ursprung haben in COPD (chronisch obstruktive pulmonale Erkrankung) oder α1-Proteinase-Inhibitor-Mangel.

Bevorzugt ist ferner die Verwendung der kristallinen und enantiomerenreinen Verbindung der Formel **1** zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die Verwendung der kristallinen und enantiomerenreinen Verbindung der Formel **1** zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung der kristallinen und enantiomerenreinen Verbindung der Formel **1** zur Herstellung eines Arzneimittels zur Behandlung von Zystischer Fibrose bzw. Mukoviszidose.

Bevorzugt ist ferner die Verwendung der kristallinen und enantiomerenreinen Verbindung der Formel **1** zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung der kristallinen und enantiomerenreinen Verbindung der Formel **1** zur Herstellung eines Arzneimittels zur Behandlung von Bronchiektasen.

Bevorzugt ist ferner die Verwendung der kristallinen und enantiomerenreinen Verbindung der Formel **1** zur Herstellung eines Arzneimittels zur Behandlung von ARDS (adult respiratory distress syndrom).

Bevorzugt ist ferner die Verwendung der kristallinen und enantiomerenreinen Verbindung der Formel **1** zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der kristallinen und enantiomerenreinen Verbindung der Formel **1** zur Herstellung eines Arzneimittels zur Behandlung von Asthma oder COPD. Von besonderer Bedeutung ist ferner die vorstehend genannte Verwendung der kristallinen und enantiomerenreinen Verbindung der Formel **1** zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Ferner betrifft die vorliegende Erfindung ein Verfahren zur Behandlung der vorstehend genannten Erkrankungen, dadurch gekennzeichnet, dass eine oder mehrere der vorstehend genannten kristallinen und enantiomerenreinen Verbindung der Formel **1** in therapeutisch wirksamen Mengen appliziert werden. Die vorliegende Erfindung betrifft bevorzugt Verfahren zur Behandlung von Asthma oder COPD, dadurch gekennzeichnet, dass vorstehend genannte kristalline und enantiomerenreine Verbindung der Formel **1** in therapeutisch wirksamen Mengen einmal täglich appliziert werden.

Zur Applikation sind Arzneimittelzusammensetzungen geeignet, die die kristalline, enantiomerenreine Verbindung **1** in einer Inhalationslösung oder einer inhalativ applizierbaren Pulverformulierung enthalten. Weiterhin geeignet sind Arzneimittelzusammensetzungen, die die erfindungsgemäße kristalline, enantiomerenreine Verbindung **1** und weiteren Wirkstoff enthalten, eine oder mehrere Verbindungen, die ausgewählt sind aus den Klassen der Anticholinergika, Corticosteroiden, PDE4-Inhibitoren, LTD4-Antagonisten, EGFR-Hemmern, Dopamin-Agonisten, H1-Antihistaminika und P13-Kinase Inhibitoren oder zwei- oder dreifach Kombinationen davon.

Ferner betrifft die vorliegende Erfindung ein Verfahren zur Behandlung der vorstehend genannten Erkrankungen, dadurch gekennzeichnet, dass vorstehend genannte kristalline, enantiomerenreine Verbindung der Formel **1** in therapeutisch wirksamen Mengen appliziert werden. Die vorliegende Erfindung betrifft bevorzugt Verfahren zur Behandlung von Asthma oder COPD, dadurch gekennzeichnet, dass vorstehend genannte kristalline, enantiomerenreine Verbindung der Formel **1** in therapeutisch wirksamen Mengen einmal täglich appliziert werden.

Geeignete Anwendungsformen zur Applikation der kristallinen, enantiomerenreinen Verbindung der Formel **1** sind beispielsweise Tabletten, Kapseln, Zäpfchen, Pulver etc. Der Anteil der pharmazeutisch wirksamen Verbindung sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.
Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Bei der erfindungsgemäß besonders bevorzugten Verwendung der kristallinen und enantiomerenreinen Verbindung gemäß Formel **1** zur Therapie von Atemwegserkrankungen werden besonders bevorzugt inhalativ applizierbare Darreichungsformen bzw. pharmazeutische Formulierungen eingesetzt. Als inhalierbare Darreichungsformen kommen Inhalationspulver und treibgashaltige Dosieraerosole in Betracht.

Die erfindungsgemäß besonders bevorzugt in kristalliner, enantiomerenreiner Form zur Anwendung gelangenden Verbindung der Formel **1** wird bevorzugt zur Herstellung von Inhalationspulvern eingesetzt. Erfindungsgemäß einsetzbare Inhalationspulver können die kristalline, enantiomerenreine Verbindung der Formel **1** entweder allein oder im Gemisch mit geeigneten physiologisch unbedenklichen Hilfsstoffen enthalten.
Sind die Wirkstoffe im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.
Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff, vorzugsweise mit einer mittleren Teilchengröße von 0,5 bis 10µm, besonders bevorzugt von 1 bis 5µm, dem Hilfsstoff beziehungsweise der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können im Treibgas die kristalline, enantiomerenreine Verbindungen **1** in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannter Inhalatoren (MDIs = metered dose inhalers) appliziert werden.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel bereits bei Dosen im µg-Bereich durch eine hohe Wirksamkeit aus. Auch oberhalb des µg-Bereichs, lassen sich die Verbindungen der Formel sinnvoll einsetzen. Die Dosierung kann dann beispielsweise auch im Milligrammbereich liegen.

Ein weiterer Aspekt der vorliegenden Erfindung betrifft die vorstehend genannten pharmazeutischen Formulierungen, gekennzeichnet durch einen Gehalt der Verbindung der Formel 1 als solche, besonders bevorzugt die vorstehend genannten inhalativ applizierbaren pharmazeutischen Formulierungen.

Die nachfolgenden Formulierungsbeipiele illustrieren die vorliegende Erfindung, ohne sie jedoch in ihrem Umfang zu beschränken:
A)

| Tabletten | pro Tablette |
|---|---|
| Wirkstoff | 100 mg |
| Milchzucker | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.
B)

| Tabletten | pro Tablette |
|---|---|
| Wirkstoff | 80 mg |
| Milchzucker | 55 mg |
| Maisstärke | 190 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.
C)

| Dosieraerosol | |
|---|---|
| Wirkstoff | 0,005 |
| Sorbitantrioleat | 0,1 |
| TG134a : TG227 2:1 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).
D)

| Inhalationspulver | |
|---|---|
| Wirkstoff | 12 µg |
| Lactose Monohydrat | ad 10 mg |

Die Herstellung des Inhalationspulvers erfolgt in üblicher Art und Weise durch Mischen der einzelnen Bestandteile.
E)

| Lösungen (in mg/100ml) | |
|---|---|
| Wirkstoff | 333.3 mg |
| Benzalkoniumchlorid | 10.0 mg |
| EDTA | 50.0 mg |
| HCl (1n) | ad pH 3.4 |

Diese Lösung kann in üblicher Art und Weise hergestellt werden.
F)

| Ampullenlösung | |
|---|---|
| Wirkstoff | 50 mg |
| Natriumchlorid | 50 mg |
| Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

### EXPERIMENTELLER TEIL

Die Verbindung **2** in Form ihrer Base ist aus WO-A-2007/054484 bekannt:

Zur Synthese von **2** sei hiermit auf das Beispiel 1d aus WO-A-2007/054484 im Zusammenhang mit der in WO-A-2007/054484 beschrieben Synthese zur Darstellung des optisch-reinen R-Enantiomers N-(5-{(R)-2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid gemäß der Formel **2** verwiesen.

### Erfindungsgemäße Darstellung der kristallinen, enantiomerenreinen Verbindung 1 mit einem Schmelzpunkt von 215°C:

### Beispiel 1:

30 g der Verbindung **2** werden in 345 mL Acetonitril und 16.5 mL Wasser suspendiert. Bei 16-18°C werden vorsichtig 6,8 g 30%ige Salzsäure zugegeben, darauf folgend weitere 45mL Acetonitril. Die Lösung wird auf 65°C erwärmt, es wird Aktivkohle zugegeben und filtriert.
Der Filterkuchen wird mit 105 mL Acetonitril gewaschen und das Filtrat refluxiert.
Die Lösung wird mittels Destillation eingeengt, wobei **1** auskristallisiert. Weitere 180 mL Acetonitril werden zugegeben und die Destillation wird fortgesetzt. Sobald 250 mL des Lösemittels abdestilliert sind, wird die Suspension auf 10°C abgekühlt und die ausgefallenen Kristalle werden abfiltriert und mit 2 x 60 mL kaltem Acetonitril gewaschen. Daraufhin werden die Kristalle bei 50°C getrocknet. Die Ausbeute beträgt 29g (91%).

### Beispiel 2:

50 g der Verbindung **2** werden in 575 mL n-Butyl-Alkohol suspendiert. Dieser Suspension werden bei Raumtemperatur vorsichtig 6,8 g 30%ige Salzsäure zugegeben, gefolgt von weiteren 20 mL Wasser und weiteren 75 mL n-Butyl-Alkohol. Die verbleibende Suspension wird auf 80°C erwärmt und die dadurch entstehende Lösung wird über Aktivkohle abfiltriert. Der Filterkuchen wird mit weiteren 225 mL n-Butyl-Alkohol gewaschen. Ein Teil des Lösemittels wird bei dieser Temperatur abdestilliert bei gleichzeitigen Anlegen eines schwachen Vakuums. Dabei ist ein Auskristallisieren von **1** zu beobachten. Nachdem 150 mL des Lösemittels entfernt ist, wird die verbleibende Suspension auf 10°C abgekühlt und die ausgefallenen Kristalle werden abfiltriert und mit 2 x 100 mL kaltem n-Butyl-Alkohol gewaschen. Daraufhin werden die Kristalle bei 50°C getrocknet. Die Ausbeute beträgt 45g (84%).

Alternativ ist auch folgende Darstellung der erfindungsgemäßen Verbindung **1** möglich:

### Beispiel 3:

2.05 mmol der freien Base der Verbindung der Formel **2** werden in 7 mL Aceton suspendiert und mit 506 µl (2.02 mmol) 4 molarer Salzsäure in 550 µl Aceton versetzt, woraufhin eine klare Lösung entsteht. Nach Zugabe einer Kristallisationshilfe werden insgesamt 7 mL Diethylether zugetropft. Der ausfallende Feststoff wird nach 3 Stunden abgetrennt, mit Diethylether gewaschen und bei 40°C getrocknet. Anschließend wird der Feststoff in Acetonitril unter Zugabe von wenigen Tropfen Wasser umkristallisiert, filtriert und wieder mit Diethylether gewaschen. Ausbeute: 46%, Schmelzpunkt: 162 ± 3 °C.

500 mg dieser Zwischenstufe wird in 5 ml Acetonitril und 0,2 mL Wasser bei 60°C gelöst. Durch Zugabe von Kristallen, wie sie beispielweise gemäß Beispiel 1 oder Beispiel 2 erhältlich sind, wird das Ausfällen der Kristalle induziert. Die Mischung wird auf Raumtemperatur abgekühlt und weitere 4 Stunden bei dieser Temperatur gerührt. Die ausgefallenen Kristalle werden abfiltriert, mit 1 mL Acetonitril 2 x 1 mL Butylmethylether gewaschen und bei 40°C getrocknet. Die Ausbeute der Verbindung gemäß Formel **1** beträgt 460 mg (92%).

### Beispiel 4:

2.05 mmol der freien Base der Verbindung der Formel **2** werden in 7 mL Aceton suspendiert und mit 506 µl (2.02 mmol) **4** molarer Salzsäure in 550 µl Aceton versetzt, woraufhin eine klare Lösung entsteht. Nach Zugabe einer Kristallisationshilfe werden insgesamt 7 mL Diethylether zugetropft. Der ausfallende Feststoff wird nach 3 Stunden abgetrennt, mit Diethylether gewaschen und bei 40°C getrocknet. Anschließend wird der Feststoff in Acetonitril unter Zugabe von wenigen Tropfen Wasser umkristallisiert, filtriert und wieder mit Diethylether gewaschen. Ausbeute: 46%, Schmelzpunkt: 162 ± 3 °C.

500 mg dieser Zwischenstufe wird in 5 mL Acetonitril suspendiert (optional kann 0,05 mL Wasser dieser Suspension zugesetzt werden) und 20 Stunden bei 40°C gerührt. Danach wird die Mischung auf Raumtemperatur abgekühlt. Die ausgefallenen Kristalle werden abfiltriert, mit 1 mL Acetonitril 2 x 1 mL Butylmethylether gewaschen und bei 40°C getrocknet. Die Ausbeute der Verbindung gemäß Formel **1** beträgt 480 mg (96%).

### RÖNTGENPULVERDIAGRAMM (ABBILDUNG 1)

### Parameter des zur Messung verwendeten Röntgenpulverdiffraktometer:

STOE Stadi P Röntgenpulverdiffraktometer mit ortsempfindlichem Detektor in Transmissionsmodus mit gebogenem Germanium (111) Primärmonochromator verwendete Wellenlänge: CuK_{α1} mit λ = 1.540598 Å; Leistungsaufnahme der Röntgenröhre: 40 kV, 40 mA; Aufnahmebereich: 3 - 40 °2Θ.

Zur Indizierung der Peaks des Röntgenpulverdiagramms wurde die Software TREOR (Teil des STOE Stadi P Software Paketes) verwendet.

### THERMOANALYSE (DSC/TG - DIAGRAMM, ABBILDUNG 2)

### Technische Daten zum verwendeten Thermoanalysegerät - DSC:

DSC 822 von der Fa. Mettler Toldeo; Aufheizrate: 10 K/min; Tiegeltyp: perforierte Aluminiumtiegel; Atmosphäre: N₂, 80 ml/min Flussrate; Einwaage: 3-10 mg.

### Technische Daten zum verwendeten Thermoanalysegerät - TG:

TGA/SDTA 851 von der Fa. Mettler Toledo mit IR-Kopplung (Nicolet FT-IR 4700) zur Analyse der ausgeheizten flüchtigen Anteile; Aufheizrate: 10 K/min; Tiegeltyp: offener Aluminiumoxidtiegel; Atmosphäre: N₂, 20 ml/min Flussrate; Einwaage: 15-25 mg.

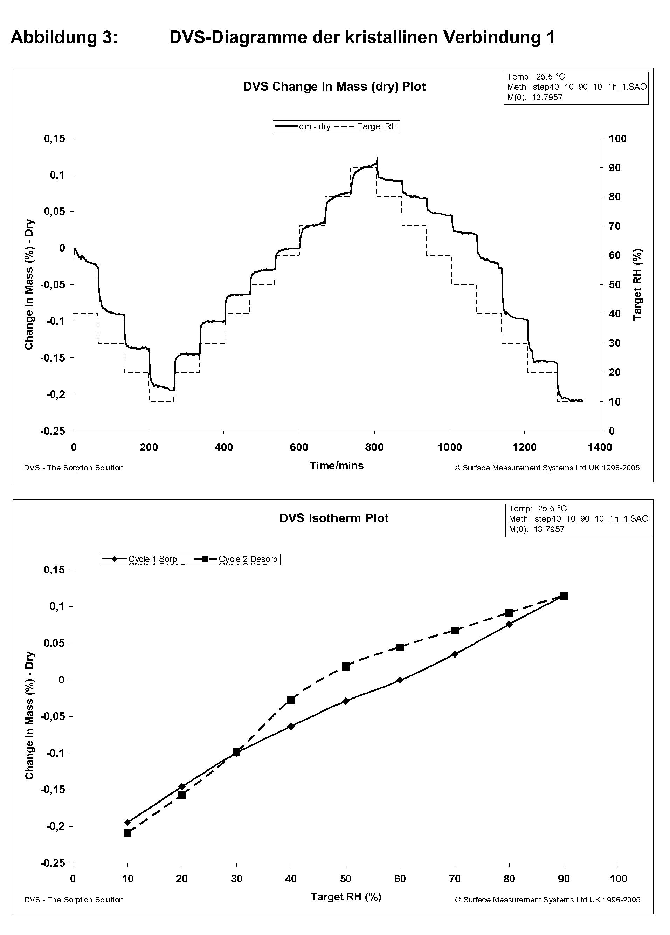

### SORPTIONSPROFIL (DSV-DIAGRAMM, KINETIK- & ISOTHERM-PLOT, ABBILDUNG 3)

### Technische Daten zur verwendeten Sorptionswaage:

DVS-1 von der Fa. Surface Measurement Systems (= SMS) zur Analyse des hygroskopischen Verhaltens; Feuchteprofil von 10 - 90 % r.F.in 10 % Schritten, wobei ein Sorptions- sowie auch ein Desorptionsprofil aufgenommen wird, Einwaage: 10 - 20 mg

### KOMBINATIONEN

Die kristalline und enantiomerenreine Verbindung der Formel 1 kann allein oder in Kombination mit anderen Wirkstoffen zur Anwendung gelangen. Gegebenenfalls kann die kristalline und enantiomerenreine Verbindung der Formel 1 auch in Kombination mit **W** eingesetzt werden, worin **W** einen pharmakologisch, aktiven Wirkstoff darstellt und (beispielsweise) ausgewählt ist, aus der Gruppe bestehend aus Anticholinergika, Corticosteroiden, PDE4-Inhibitoren, LTD4-Antagonisten, EGFR-Hemmern, Dopamin-Agonisten, H1-Antihistaminika und PI3-Kinase Inhibitoren. Weiterhin können zwei- oder dreifach Kombinationen von **W** mit der kristallinen und enantiomerenreinen Verbindung der Formel 1 kombiniert werden. Beispielhaft genannte Kombinationen von **W** wären:
- **W** stellt ein Anticholinergikum dar, kombiniert mit einem Betamimetikum, Corticosteroid, PDE4-Inhibitor, EGFR-Hemmer oder LTD4-Antagonisten,
- **W** stellt ein Corticosteroid dar, kombiniert mit einem PDE4-Inhibitor, EGFR-Hemmer oder LTD4-Antagonisten
- **W** stellt ein PDE4-Inhibitor dar, kombiniert mit einem EGFR-Hemmer oder LTD4-Antagonisten
- **W** stellt ein EGFR-Hemmer dar, kombiniert mit einem LTD4-Antagonisten.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, lpratropiumsalzen, bevorzugt das Bromidsalz, Glycopyrroniumsalzen, bevorzugt das Bromidsalz, Aclidiniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodid und Methansulfonat besonders bevorzugt. Weiterhin genannte Verbindungen sind 2,2-Diphenylpropionsäuretropenolester-methobromid; 2,2-Diphenylpropionsäurescopinester-Methobromid; 2-Fluor-2,2-Diphenylessigsäurescopinester-Methobromid; 2-Fluor-2,2-Diphenylessigsäuretropenolester-Methobromid; 3,3',4,4'-Tetrafluorbenzilsäuretropenolester-Methobromid; 3,3',4,4'-Tetrafluorbenzilsäurescopinester-Methobromid; 4,4'-Difluorbenzilsäuretropenolester-Methobromid; 4,4'-Difluorbenzilsäurescopinester-Methobromid; 3,3'-Difluorbenzilsäuretropenolester-Methobromid; 3,3'-Difluorbenzilsäurescopinester-Methobromid; 9-Hydroxy-fluoren-9-carbonsäuretropenolester-Methobromid; 9-Fluorfluoren-9-carbonsäuretropenolester-Methobromid; 9-Hydroxy-fluoren-9-carbonsäurescopinester-Methobromid; 9-Fluor-fluoren-9-carbonsäurescopinester Methobromid; 9-Methyl-fluoren-9-carbonsäuretropenolesterMethobromid; 9-Methylfluoren-9-carbonsäurescopinesterMethobromid; Benzilsäurecyclopropyltropinester-Methobromid; 2,2-Diphenylpropionsäurecyclopropyltropinester-Methobromid; 9-Hydroxyxanthen-9-carbonsäurecyclopropyltropinesterMethobromid; 9-Methyl-fluoren-9-carbonsäurecyclopropyltropinester-Methobromid; 9-Methyl-xanthen-9-carbonsäurecyclopropyltropinester-Methobromid; 9-Hydroxy-fluoren-9-carbonsäurecyclopropyltropinester-Methobromid; 4,4'-Difluorbenzilsäuremethylestercyclopropyltropinester-Methobromid; 9-Hydroxy-xanthen-9-carbonsäuretropenolester-Methobromid; 9-Hydroxy-xanthen-9-carbonsäurescopinester Methobromid; 9-Methyl-xanthen-9-carbonsäuretropenolester-Methobromid; 9-Methylxanthen-9-carbonsäurescopinesterMethobromid; 9-Ethyl-xanthen-9-carbonsäuretropenolester Methobromid; 9-Difluormethyl-xanthen-9-carbonsäuretropenolester-Methobromid; 9-Hydroxymethyl-xanthen-9-carbonsäurescopinester -Methobromid.

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Deflazacort, RPR-106541, NS-126, ST-26 und 6,9-Difluoro-17-[(2-furanylcarbonyl)oxy]-11-hydroxy-16-methyl-3-oxo-androsta-1,4-dien-17-carbothionsäure (S)-fluoromethylester; 6,9-Difluoro-11-hydroxy-16-methyl-3-oxo-17-propionyloxy-androsta-1,4-dien-17-carbothionsäure (S)-(2-oxo-tetrahydro-furan-3S-yl)ester; Etiprednol-dichloroacetat gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und N-(3,5-Dichloro-1-oxo-pyridin-4-yl)-4-difluoromethoxy-3-cyclopropylmethoxybenzamid; (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-diisopropylbenzamid; (R)-(+)-1-(4-Bromobenzyl)-4-[(3-cyclopentyloxy)-4-methoxyphenyl]-2-pyrrolidon; 3-(Cyclopentyloxy-4-methoxyphenyl)-1-(4-N'-[N-2-cyano-S-methylisothioureido]benzyl)-2-pyrrolidon; cis[4-Cyano-4-(3-cyclopentyloxy-4-methoxyphenyl)cyclohexan-1-carbonsäure]; 2-carbomethoxy-4-cyano-4-(3-cyclopropylmethoxy-4-difluoromethoxyphenyl)cyclohexan-1-on; cis[4-Cyano-4-(3-cyclopropylmethoxy-4-difluoromethoxyphenyl)cyclohexan-1-ol]; (R)-(+)-Ethyl[4-(3-cyclopentyloxy-4-methoxyphenyl)pyrrolidin-2-yliden]acetat; (S)-(-)-Ethyl[4-(3-cyclopentyloxy-4-methoxyphenyl)pyrrolidin-2-yliden]acetat; 9-Cyclopentyl-5,6-dihydro-7-ethyl-3-(2-thienyl)-9*H*-pyrazolo[3,4-c]-1,2,4-triazolo[4,3-a]pyridin; 9-Cyclopentyl-5,6-dihydro-7-ethyl-3-(tert-butyl)-9H-pyrazolo[3,4-c]-1,2,4-triazolo[4,3-a]pyridin gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und 1-(((R)-(3-(2-(6,7-Difluoro-2-quinolinyl)ethenyl)phenyl)-3-(2-(2- hydroxy-2-propyl)phenyl)thio)methylcyclopropan-essigsäure; 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)-propyl)thio)methyl)cyclopropanessigsäure; [2-[[2-(4-tert-Butyl-2-thiazolyl)-5-benzofuranyl]oxymethyl]phenyl]essigsäure gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und 4-[(3-Chior-4-fluorphenyl)amino]-6-{[4-(morpholin-4-yl)-1-oxo-2-buten-1-yl]amino}-7-cyclopropylmethoxy-chinazolin; 4-[(3-Chlor-4-fluorphenyl)amino]-6-{[4-(N,N-diethylamino)-1-oxo-2-buten-1-yl]amino}-7-cyclopropylmethoxy-chinazolin; 4-[(3-Chlor-4-fluorphenyl)amino]-6-{[4-(N,N-dimethylamino)-1-oxo-2-buten-1-yl]amino}-7-cyclopropylmethoxy-chinazolin; 4-[(R)-(1-Phenyl-ethyl)amino]-6-{[4-(morpholin-4-yl)-1-oxo-2-buten-1-yl]amino}-7-cyclopentyloxy-chinazolin; 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin; 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin; 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin; 4-[(3-Chlor-4-fluor-phenyl)amino]-6-[2-((S)-6-methyl-2-oxo-morpholin-4-yl)-ethoxy]-7-methoxy-chinazolin; 4-[(3-Chlor-4-fluorphenyl)amino]-6-((4-[N-(2-methoxy-ethyl)-N-methyl-amino]-1-oxo-2-buten-1-yl}amino)-7-cyclopropylmethoxy-chinazolin; 4-[(3-Chlor-4-fluorphenyl)amino]-6-{[4-(N,N-dimethylamino)-1-oxo-2-buten-1-yl]amino}-7-cyclopentyloxy-chinazolin; 4-[(R)-(1-Phenyl-ethyl)amino]-6-}[4-(N,N-bis-(2-methoxy-ethyl)-amino)-1-oxo-2-buten-1-yl]amino}-7-cyclopropylmethoxy-chinazolin; 4-[(R)-(1-Phenylethyl)amino]-6-({4-[N-(2-methoxy-ethylo)-N-ethyl-amino]-1-oxo-2-buten-1-yl}amino)-7-cyclopropylmethoxy-chinazolin; 4-[(R)-(1-Phenyl-ethyl)amino]-6-((4-[N-(2-methoxy-ethyl)-N-methyl-amino]-1-oxo-2-buten-1-yl}amino)-7-cyclopropylmethoxy-chinazolin; 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin; 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin; 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin; 4-[(3-Chlor-4-fluorphenyl)amino]-6-((4-[N-(2-methoxy-ethyl)-N-methyl-amino]-1-oxo-2-buten-1-yl}amino)-7-cyclopentyloxy-chinazolin; 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin; 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin; 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin; 4-[(3-Ethinyl-phenyl)amino]-6,7-bis-(2-methoxy-ethoxy)-chinazolin; 4-[(3-Chlor-4-fluorphenyl)amino]-7-[3-(morpholin-4-yl)-propyloxy]-6-[(vinylcarbonyl)amino]-chinazolin; 4-[(R)-(1-Phenyl-ethyl)amino]-6-(4-hydroxy-phenyl)-7H-pyrrolo[2,3-d]pyrimidin; 3-Cyano-4-[(3-chlor-4-fluorphenyl)amino]-6-{[4-(N,N-dimethylamino)-1-oxo-2-buten-1-yl]amino}-7-ethoxy-chinolin; 4-{[3-Chlor-4-(3-fluor-benzyloxy)-phenyl]amino}-6-(5-{[(2-methansulfonyl-ethyl)amlno]methyl}-furan-2-yl)chinazolin; 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin; 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin; 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin; 4-[(3-Ethinyl-phenyl)amino]-6-{[4-(5,5-dimethyl-2-oxo-morpholin-4-yl)-1-oxo-2-buten-1-yl]amino}-chinazolin; 4-[(3-Chlor-4-fluor-phenyl)amino]-6-[2-(2,2-dimethyl-6-oxo-morpholin-4-yl)-ethoxy]-7-methoxy-chinazolin; 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin; 4-[(3-Chlor-4-fluorphenyl)amino]-7-[2-(2,2-dimethyl-6-oxo-morpholin-4-yl)-ethoxy]-6-[(S)-(tetrahydrofuran-2-yl)methoxy]-chinazolin; 4-[(3-Chlor-4-fluor-phenyl)amino]-6-(2-[4-(2-oxo-morpholin-4-yl)-piperidin-1-yl]-ethoxy}-7-methoxy-chinazolin; 4-[(3-Chlor-4-fluor-phenyl)amino]-6-[1-(tert.-butyloxycarbonyl)-piperidin-4-yloxy]-7-methoxy-chinazolin; 4-[(3-Chlor-4-fluorphenyl)amino]-6-(trans-4-amino-cyclohexan-1-yloxy)-7-methoxy-chinazolin; 4-[(3-Chlor-4-fluor-phenyl)amino]-6-(trans-4-methansulfonylamino-cyclohexan-1-yloxy)-7-methoxy-chinazolin; 4-[(3-Chlor-4-fluor-phenyl)amino]-6-(tetrahydropyran-3-yloxy)-7-methoxy-chinazolin; 4-[(3-Chlor-4-fluor-phenyl)amino]-6-(1-methyl-piperidin-4-yloxy)-7-methoxy-chinazolin; 4-[(3-Chlor-4-fluor-phenyl)amino]-6-{1-[(morpholin-4-yl)carbonyl]-piperidin-4-yloxy}-7-methoxy-chinazolin; 4-[(3-Chlor-4-fluor-phenyl)amino]-6-{1-[(methoxymethyl)carbonyl]-piperidin-4-yloxy}-7-methoxy-chinazolin; 4-[(3-Chlor-4-fluorphenyl)amino]-6-(piperidin-3-yloxy)-7-methoxy-chinazolin; 4-[(3-Chlor-4-fluorphenyl)amino]-6-[1-(2-acetylamino-ethyl)-piperidin-4-yloxy]-7-methoxy-chinazolin; 4-[(3-Chlor-4-fluor-phenyl)amino]-6-(tetrahydropyran-4-yloxy)-7-ethoxy-chinazolin; 4-[(3-Chlor-4-fluor-phenyl)amino]-6-((S)-tetrahydrofuran-3-yloxy)-7-hydroxy-chinazolin; 4-[(3-Chlor-4-fluor-phenyl)amino]-6-(tetrahydropyran-4-yloxy)-7-(2-methoxy-ethoxy)-chinazolin; 4-[(3-Chlor-4-fluor-phenyl)amino]-6-{trans-4-[(dimethylamino)sulfonylamino]-cyclohexan-1-yloxy}-7-methoxy-chinazolin; 4-[(3-Chlor-4-fluor-phenyl)amino]-6-{trans-4-[(morpholin-4-yl)carbonylamino]-cyclohexan-1-yloxy}-7-methoxy-chinazolin; 4-[(3-Chlor-4-fluorphenyl)amino]-6-{trans-4-[(morpholin-4-yl)sulfonylamino]-cyclohexan-1-yloxy}-7-methoxy-chinazolin; 4-[(3-Chlor-4-fluor-phenyl)amino]-6-(tetrahydropyran-4-yloxy)-7-(2-acetylamino-ethoxy)-chinazolin; 4-[(3-Chlor-4-fluor-phenyl)amino]-6-(tetrahydropyran-4-yloxy)-7-(2-methansulfonylamino-ethoxy)-chinazolin; 4-[(3-Chlor-4-fluor-phenyl)amino]-6-{1-[(piperidin-1-yl)carbonyl]-piperidin-4-yloxy}-7-methoxy-chinazolin; 4-[(3-Chlor-4-fluorphenyl)amino]-6-(1-aminocarbonylmethyl-piperidin-4-yloxy)-7-methoxy-chinazolin; 4-[(3-Chlor-4-fluor-phenyl)amino]-6-(cis-4-{N-[(tetrahydropyran-4-yl)carbonyl]-N-methy-amino}-cyclohexan-1-yloxy)-7-methoxy-chinazolin; 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin; 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin; 4-[(3-Chlor-4-fluor-phenyl)amino]-6-(trans-4-ethansulfonylamino-cyclohexan-1-yloxy)-7-methoxy-chinazolin; 4-[(3-Chlor-4-fluorphenyl)amino]-6-(1-methansulfonyl-piperidin-4-yloxy)-7-ethoxy-chinazolin; 4-[(3-Chlor-4-fluor-phenyl)amino]-6-(1-methansulfonyl-piperidin-4-yloxy)-7-(2-methoxy-ethoxy)-chinazolin; 4-[(3-Chlor-4-fluor-phenyl)amino]-6-[1-(2-methoxy-acetyl)-piperidin-4-yloxy]-7-(2-methoxy-ethoxy)-chinazolin; 4-[(3-Chlor-4-fluor-phenyl)amino]-6-(cis-4-acetylaminocyclohexan-1-yloxy)-7-methoxy-chinazolin; 4-[(3-Ethinyl-phenyl)amino]-6-[1-(tert.-butyloxycarbonyl)-piperidin-4-yloxy]-7-methoxy-chinazolin; 4-[(3-Ethinyl-phenyl)amino]-6-(tetrahydropyran-4-yloxy]-7-methoxy-chinazolin; 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin; 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin; 4-[(3-Chlor-4-fluor-phenyl)amino]-6-{cis-4-[(morpholin-4-yl)carbonylamino]-cyclohexan-1-yloxy}-7-methoxy-chinazolin; 4-[(3-Chlor-4-fluor-phenyl)amino]-6-{1-[2-(2-oxopyrrolidin-1-yl)ethyl]-piperidin-4-yloxy}-7-methoxy-chinazolin; 4-[(3-Chlor-4-fluor-phenyl)amino]-6-{1-[(morpholin-4-yl)carbonyl]-piperidin-4-yloxy}-7-(2-methoxy-ethoxy)-chinazolin; 4-[(3-Ethinyl-phenyl)amino]-6-(1-acetyl-piperidin-4-yloxy)-7-methoxy-chinazolin; 4-[(3-Ethinylphenyl)amino]-6-(1-methyl-piperidin-4-yloxy)-7-methoxy-chinazolin; 4-[(3-Ethinylphenyl)amino]-6-(1-methansulfonyl-piperidin-4-yloxy)-7-methoxy-chinazolin; 4-[(3-Chlor-4-fluor-phenyl)amino]-6-(1-methyl-piperidin-4-yloxy)-7(2-methoxy-ethoxy)-chinazolin; 4-[(3-Chlor-4-fluor-phenyl)amino]-6-(1-isopropyloxycarbonyl-piperidin-4-yloxy)-7-methoxy-chinazolin; 4-[(3-Chlor-4-fluor-phenyl)amino]-6-(cis-4-methylamino-cyclohexan-1-yloxy)-7-methoxy-chinazolin; 4-[(3-Chlor-4-fluor-phenyl)amino]-6-(cis-4-[N-(2-methoxy-acetyl)-N-methyl-amino]-cyclohexan-1-yloxy}-7-methoxy-chinazolin; 4-[(3-Ethinyl-phenyl)amino]-6-(piperidin-4-yloxy)-7-methoxy-chinazolin; 4-[(3-Ethinyl-phenyl)amino]-6-[1-(2-methoxyacetyl)-piperidin-4-yloxy]-7-methoxy-chinazolin; 4-[(3-Ethinyl-phenyl)amino]-6-{1-[(morpholin-4-yl)carbonyl]-piperidin-4-yloxy}-7-methoxy-chinazolin; 4-[(3-Chlor-4-fluorphenyl)amino]-6-{1-[(cis-2,6-dimethyl-morpholin-4-yl)carbonyl]-piperidin-4-yloxy}-7-methoxy-chinazolin; 4-[(3-Chlor-4-fluor-phenyl)amino]-6-{1-[(2-methyl-morpholin-4-yl)carbonyl]-piperidin-4-yloxy}-7-methoxy-chinazolin; 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin; 4-[(3-Chlor-4-fluor-phenyl)amino]-6-{1-[(N-methyl-N-2-methoxyethylamino)carbonyl]-piperidin-4-yloxy}-7-methoxy-chinazolin; 4-[(3-Chlor-4-fluorphenyl)amino]-6-(1-ethyl-piperidin-4-yloxy)-7-methoxy-chinazolin; 4-[(3-Chlor-4-fluorphenyl)amino]-6-{1-[(2-methoxyethyl)carbonyl]-piperidin-4-yloxy}-7-methoxy-chinazolin; 4-[(3-Chlor-4-fluor-phenyl)amino]-6-{1-[(3-methoxypropyl-amino)-carbonyl]-piperidin-4-yloxy}-7-methoxy-chinazolin; 4-[(3-Chlor-4-fluor-phenyl)amino]-6-[cis-4-(N-methansulfonyl-N-methyl-amino)-cyclohexan-1-yloxy]-7-methoxy-chinazolin; 4-[(3-Chlor-4-fluor-phenyl)amino]-6-[cis-4-(N-acetyl-N-methyl-amino)-cyclohexan-1-yloxy]-7-methoxy-chinazolin; 4-[(3-Chlor-4-fluor-phenyl)amino]-6-(trans-4-methylamino-cyclohexan-1-yloxy)-7-methoxy-chinazolin; 4-[(3-Chlor-4-fluor-phenyl)amino]-6-[trans-4-(N-methansulfonyl-N-methyl-amino)-cyclohexan-1-yloxy]-7-methoxy-chinazolin; 4-[(3-Chlor-4-fluor-phenyl)amino]-6-(trans-4-dimethylamino-cyclohexan-1-yloxy)-7-methoxy-chinazolin; 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin; 4-[(3-Chlor-4-fluorphenyl)amino]-6-[2-(2,2-dimethyl-6-oxo-morpholin-4-yl)-ethoxy]-7-[(S)-(tetrahydrofuran-2-yl)methoxy]-chinazolin; 4-[(3-Chlor-4-fluor-phenyl)amino]-6-(1-methansulfonyl-piperidin-4-yloxy)-7-methoxy-chinazolin; 4-[(3-Chlor-4-fluor-phenyl)amino]-6-(1-cyano-piperidin-4-yloxy)-7-methoxy-chinazolin gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

## Patentansprüche

**1.** Kristallines Hydrochlorid-Salz von N-(5-{(R)-2-[3-(4,4-Diethyl-2-oxo-4H-benzo[d][1,3]oxazin-1-yl)-1,1-Dimethyl-propylamino]-1-hydroxy-ethyl}-2-hydroxyphenyl)-methansulfonamid der Verbindung **1**

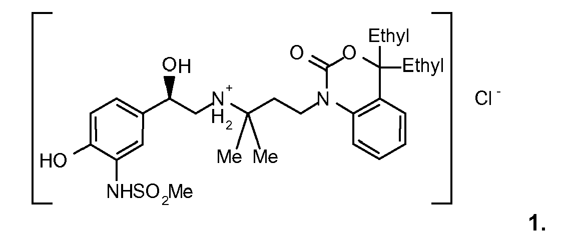

**2.** Kristallines Hydrochlorid-Salz der Verbindung **1 dadurch gekennzeichnet, dass** es bei 215±3°C unter Zersetzung schmilzt.

**3.** Kristallines Hydrochlorid-Salz der Verbindung **1** nach einem der Ansprüche 1 bis 2, **dadurch gekennzeichnet dass** es bei d = 10,03 Å; 5,76 Å; 4,82 Å; 4,36 Å Röntgenreflexe aufweist.

**4.** Kristallines Hydrochlorid-Salz der Verbindung **1** nach einem der Ansprüche 1 bis 3, **dadurch gekennzeichnet dass** es bei d = 10,03 Å; 6,10 Å; 5,76 Å; 4,82 Å; 4,36 Å; 4,26 Å; 4,13 Å; 4,09 Å Röntgenreflexe aufweist.

**5.** Kristallines Hydrochlorid-Salz der Verbindung **1** nach einem der Ansprüche 1 bis 4, **dadurch gekennzeichnet, dass** dessen Kristallstruktur eine einfache orthorhombische Zelle (Raumgruppe P2₁2₁2₁) mit folgenden Zellkonstanten: a = 30.58±0.05(16) Å, b = 13.29±0.05 (8) Å, c = 7.04±0.05 (3) Å, α = β = γ = 90°, V = 2862±5 (37) Å³ aufweist.

**6.** Kristallines Hydrochlorid-Salz der Verbindung **1** nach einem der Ansprüche 1 bis 5, **dadurch gekennzeichnet dass** es 1 Hydrochloridmolekül enthält.

**7.** Pharmazeutische Zusammensetzung, **dadurch gekennzeichnet dass** sie ein kristallines Hydrochlorid-Salz der Verbindung **1** nach einem der Ansprüche 1 bis 6 enthält.

**8.** Verwendung des kristallinen Hydrochlorid-Salz der Verbindung **1** nach einem der Ansprüche 1 bis 6 zur Herstellung einer Inhalationslösung zur Behandlung von Atemwegserkrankungen.

**9.** Verwendung des kristallinen Hydrochlorid-Salzes von N-(5-{2-[3-(4,4-diethyl-2-oxo-4H-benzo[d][1,3]oxazin-1-yl)-1,1-dimethyl-propylamino]-1-hydroxy-ethyl}-2-hydroxyphenyl)-methansulfonamide nach einem der Ansprüche 1 bis 4 zur Herstellung einer inhalativ applizierbaren Pulverformulierung zur Behandlung von Atemwegserkrankungen.

**10.** Verwendung des kristallinen Hydrochlorid-Salz der Verbindung **1** nach einem der Ansprüche 1 bis 6 zur Herstellung einer Treibgas-basierten HFA-MDI Formulierung zur Behandlung von Atemwegserkrankungen.

**11.** Arzneimittelkombinationen, die neben dem kristallinen Hydrochlorid-Salz der Verbindung **1** nach einem der Ansprüche 1 bis 6 als weiteren Wirkstoff einen oder mehrere Verbindungen enthalten, die ausgewählt sind aus den Klassen der Anticholinergika, Corticosteroide, PDE4-Inhibitoren, LTD4-Antagonisten, EGFR-Hemmern, Dopamin-Agonisten, H1-Antihistaminika und PI3-Kinase Inhibitoren oder zwei- oder dreifach Kombinationen davon.

**12.** Herstellung einer Inhalationslösung zur Behandlung von Atemwegserkrankungen, **dadurch gekennzeichnet dass** als Wirkstoff das kristalline Hydrochlorid-Salz der Verbindung **1** nach einem der Ansprüche 1 bis 6 darin gelöst wird.

**12.** Herstellung einer Inhalationslösung nach Anspruch 10, **dadurch gekennzeichnet dass** als weiterer Wirkstoff eine oder mehrere Verbindungen, ausgewählt aus den Klassen der Anticholinergika, Corticosteroide, PDE4-Inhibitoren, LTD4-Antagonisten, EGFR-Hemmern, Dopamin-Agonisten, H1-Antihistaminika und PI3-Kinase Inhibitoren oder zwei- oder dreifach Kombinationen davon, darin gelöst werden.
